# NEW EUROPEAN PATENT SPECIFICATION

(11) **EP 1 542 635 B2**
(45) Date of publication and mention of the opposition decision: **08.05.2019**
(45) Mention of the grant of the patent: 25.04.2012
(21) Application number: 03754582.9
(22) Date of filing: 15.09.2003
(51) Int. Cl.: A61F 13/42, H01H 35/42, A61F 13/20

(54) **DISPOSABLE ARTICLES HAVING A FAILURE DETECTION SYSTEM**
WEGWERFARTIKEL, DIE EIN STÖRUNGSERKENNUNGSSYSTEM HABEN
ARTICLES JETABLES PRESENTANT UN SYSTEME DE DETECTION DE DEFAILLANCE

(30) Priority: 27.09.2002 US 259093
(43) Date of publication of application: 22.06.2005
(73) Proprietor: McNeil-PPC, Inc., Skillman, New Jersey 08558 (US)
(72) Inventor: DAVID, Benoit, Washington Crossing, PA 19877 (US); HOLLIDAY, Christopher, M., Yardley, PA 19067 (US); HARTMAN, Frederick, R., Englishtown, NJ 07726 (US); GILLESPIE, Ronald, J., North Brunswick, NJ 08902 (US)
(74) Representative: Metten, Karl-Heinz
(86) International application number: PCT/US2003/028915
(87) International publication number: WO 2004/028429

(56) References cited:
- WO-A-02/078513
- WO-A1-99/33037
- GB-A- 890 310
- US-A- 341 377
- US-A- 4 754 264
- US-A- 4 799 929
- US-A- 6 063 042
- US-A- 6 063 042
- US-A- 6 097 297
- US-A1- 2002 070 864
- US-B1- 6 348 640
- US-B1- 6 908 458
- Reply 24-09-2007 to communication 14-03-2007
- Fundamental University Physics vol.II FIELDS AND WAVES. ED.4, vol. II, 1972, pages 579-580,

## Description

The present invention relates to disposable articles, and more particularly to a disposable article wetness detection system that signals the user that the disposable article is approaching capacity and it is time to change the article.

### Background of the Invention

Disposable articles, such as feminine hygiene tampons are primarily designed to absorb and/or to contain a particular amount of bodily fluids, such as menstrual fluid. The amount of menstrual fluid absorbed by a tampon can vary depending on absorbency levels. For example, in the United States, tampon absorbency can range from less than 6 grams (Junior absorbency) grams to 15-18 grams (Ultra absorbency). In order to ascertain whether a tampon has reached its absorbent capacity, the tampon must be removed and viewed, resulting in the destruction of the tampon as most women are reluctant to reinsert the tampon. In most cases, a user will remove a tampon before it has reached its absorbent capacity in order to prevent an accident wherein the absorbent capacity of the tampon is exceeded. Once the absorbent capacity is exceeded, the excess menses flows unimpeded from the vagina to soil the user's clothing.

A determinative criterion frequently used to gauge tampon replacement is the amount of time elapsed since insertion. The time elapsed criterion for changing tampons is not satisfactory for several reasons, e.g., the menstrual flow rate varies throughout the menstruating period and much adsorbent capacity of tampons is wasted due to the tendency to change before an accident occurs.

The flow variation throughout the period causes problems as to how long to wear a tampon because a user cannot establish a definite time period for which the absorbent capacity within a tampon is sufficient. Therefore, the user is in a quandary as to how long to wear specific tampons during days of heavy flow as contrasted to days of light flow.

A correlation between tampon performance during light flow versus heavy flow is difficult for the user to make. Since most users would err on the conservative side and want to be sure about protection, the typical user will prematurely remove a tampon before the absorbent capacity of the tampon has been reached. This wastes much of the absorbent capacity of the product purchased.

Patent literature has described attempts to alert a tampon user to change her tampon. Examples of these systems are disclosed in US Patent Nos. 6,348,640, 6,063,042; 5,904,671, and WO 99/17692, (all to Navot et al.). US Patent No 6,063,042 purports to disclose a system for diagnosing menstrual cycle disorders including menorrhagia by use of a vaginal device having at least one sensor, a reporter being in data communication with the sensor device and an analyzing unit. The data communication between the sensor and reporter may be by direct communication such as wire communication. Alternately, the data communication may be by remote communication. A transmitter in direct communication with the sensor device and a compatible receiver in communication with the reporter combine to provide remote communication. The transmitter and sensor require a power source, such as a battery for its operation.

US Patent Nos. 6,348,640 and 5,904,671 and WO 99/17692 purport to disclose tampon wetness detection systems having a tampon, a radio frequency identification device including a transmitter, a wetness sensor and a remote signaling device including a receiver. The detection system can provide a remote-reporter with information regarding the wetness of the tampon, its remaining capacity, and the concentration of various substances. The system has a housing insertable into a tampon, a radio frequency identification device including a transmitter, a conductive wetness sensor in electrical communication with the radio frequency identification device and a remote-reporting device including a receiver for receiving a radio signal concerning the wetness of the tampon. The radio frequency identification device can be active (requiring a self-sustained power source, such as a battery) or passive (employing a capacitor charged by a remote radio transmitter).

The present invention provides a wetness detection system in which an external device essentially "reads" the wetness of the tampon and signals the user to change the tampon prior to its soiling of the user's clothing.

None of the above examples has completely solved the problem of indicating when the disposable article should be changed in order to prevent leakage. Thus, there is a need for a failure detection system that signals the user to change the disposable article prior to soiling the user's clothing.

### Summary of the Invention

It is an object of the present invention to provide a disposable article having a failure detection system that signals the user to change the article prior to its sailing af a user's clothing.

It is another object of the present invention to provide a disposable article having a remote device that communicates with the disposable article to provide early information about the status of the disposable article.

It is another object of the present invention to provide a method of controlling aqueous vaginal fluids using a disposable article having a failure detection system.

In accordance with one aspect of the present invention a system for detecting wetness in an absorbent article in accordance with the features of claim 1 has been found

In accordance with another aspect of the present invention, a method of controlling liquid bodily exudates in accordance with the features of claim 9 has been found.

The disposable article includes an absorbent structure and at least one sensor associated with the absorbent structure. The sensor provides a variable electrical output dependent upon an amount of aqueous liquid associated with the absorbent structure.

### Brief Description of the Drawings

Fig. 1 is a simplified schematic depiction of the components of a typical sensor according to one embodiment of the present invention;
Fig. 2 is a simplified schematic depiction of a wetness detection system according to the present invention including a disposable article and a remote device;
Fig. 3 is a simplified schematic depiction of an alternate embodiment of the disposable article of Fig. 2; and
Fig. 4 is a simplified schematic depiction of an alternate embodiment of the present invention.

### Detailed Description of the invention

Further characteristics and advantages of the invention will become clear from the following detailed description, appended drawings, and non-limiting examples.

The present invention is directed to a system for detecting wetness in a disposable article that is used in contact with a user's body. The disposable articles have an absorbent structure and at least one sensor in contact with the absorbent structure. The sensor provides a variable electrical output dependent upon the amount of aqueous liquid associated with the absorbent structure. The system also includes a remote interrogating device capable of detecting changes in the variable electrical output of the at least one sensor and a reporting element coupled to the interrogating device. Examples of such articles include, without limitation, external sanitary protection articles such as sanitary napkins, pantiliners, and interlabial devices; absorbent incontinence articles such as diapers and incontinence pads and guards; internal sanitary protection articles such as tampons, collection cups and other vagina-occluding devices, and pessaries; wound care articles such as bandages; and the like.

As used herein in the specification and the claims, the term "passive interrogation" and related terms mean an interrogation system that requires no power source connected to the sensors. The sensors are read or interrogated by a separate device.

As used herein in the specification and the claims, the term "transmitter" and related terms mean a device capable of transmitting or sending relevant signals to a remote receiver. The transmitter is provided with power for operation by a power source.

As used herein the specification and the claims, the term "remote or external" and related terms refer to that part of the absorbent device that is maintained outside of the body. The remote device typically has a power source.

As used herein the specification and the claims, the term "interrogate" and related terms mean to transmit a signal to a targeted body that produces a measurable response. The response to interrogation is measurable by the remote device and provides an indication to the user as to when to change the targeted body.

Depending upon its desired use, the disposable article may absorb or block the passage of, especially, bodily fluids. If the disposable article is to absorb the bodily fluids, the absorbent structure will form a great proportion of the disposable article. If the disposable article is to block the passage of the bodily fluids, it will comprise a smaller absorbent structure that collects fluid for the sensor that may have bypassed or overflowed a device such as a vaginal collection cup, or occlude the body cavity, such as an inflatable device disclosed in Kamen et al., US Pat. No. 6,168,609 B1.

In order to better explain the present invention, it will be discussed in conjunction with an absorbent tampon. However, one of ordinary skill in the art will recognize that it is useful with other disposable articles.

Absorbent tampons are usually substantially cylindrical masses of compressed absorbent material having a central axis and a radius that defines the outer circumferential surface of the tampon. Tampons are often formed by first obtaining a shaped mass of absorbent material called a tampon blank. This blank can be in the form of a roll of sheet-like material, a segment of a continuous absorbent material, a mass of randomly or substantially uniformly oriented absorbent material, an individually prepared or cast mass of absorbent material, and the like.

In an embodiment, the tampon blank is relatively uncompressed and has a relatively low density. It is then compressed to form a product having overall dimensions less than those of the blank prior to use. The compressed tampons may have a generally uniform density throughout the tampon, or they may have regions of differing density as described in the commonly assigned applications to Friese et al., US Pat. No. 6,310,269, and Leutwyler et al., US Pat. No. 5,813,102.

Tampons also usually include a cover or some other surface treatment and a withdrawal string or other removal mechanism.

The tampon blank may be substantially enclosed by a fluid-permeable cover. Thus, the cover encloses a majority of the outer surface of the tampon. This may be achieved as disclosed in Friese, US Patent No. 4,816,100. In addition, either or both ends of the tampon may be enclosed by the cover. Of course, for processing or other reasons, some portions of the surface of the tampon may be free of the cover. For example, the insertion end of the tampon and a portion of the cylindrical surface adjacent this end may be exposed, without the cover to allow the tampon to more readily accept fluids.

Absorbent materials useful in the formation of the absorbent body include fiber, foam, superabsorbent, hydrogels, wood pulp, and the like. Preferred absorbent material for the present invention includes foam and fiber. Absorbent foams may include hydrophilic foams, foams which are readily wetted by aqueous fluids as well as foams in which the cell walls that form the foam themselves absorb fluid.

Fibers employed in the formation of the absorbent body may include regenerated cellulosic fiber, natural fibers and synthetic fibers. Preferably, the materials employed in the formation of a vaginal tampon according to the present invention include fiber, foam, hydrogels, wood pulp, and the like.

A useful, non-limiting list of useful absorbent body fibers includes natural fibers such as cotton, wood pulp, jute, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the absorbent body. Preferably, tampon fibers are rayon or cotton, and more preferably, the fibers are rayon. The fibers may have any useful cross-section.

The systems of the present invention are based on passive interrogation in which the sensor is not directly connected to a power source. The sensor provides a variable electrical output dependent upon an amount of aqueous liquid associated with tho absorbent structure. The variable electrical output may include, without limitation, frequency, voltage, current, and the like. The output may be provided by a frequency output generator or by a variable electrical component as described herein.

The remote device usually includes a transmitter, a receiver, a reporting element, and a power source. The transmitter is capable of transmitting electromagnetic energy to the target. The receiver is compatible with the transmitter and can detect altered signal strength or a phase shift from the transmission. The reposing element is useful to report information derived from the receiver to the user. This information may be used to understand the state of the disposable article. The power source (e.g., a rechargeable battery) supplies the necessary power to the remote device.

The remote device may include additional devices, including data manipulation devices such as computers, etc., to transform the data into more detailed information. While it is not critical to the operation of the system, it is useful if the remote device is reusable. The remote device may be contained in an accessory such as a buckle or jewelry, a cosmetic compact, a hand-held device, etc. The reporting element may report information through any sensory means, including without limitation, tactile (e.g., vibrations), audio (e.g., tones or a buzzer), visual (e.g., lights, color change, alphanumeric display), and the like. The information can be provided continually or as prompted by the user. It can also provide information regarding the moisture change over time at any particular sensor and the rate of absorption thereat to allow measurement of the performance of the absorbent body.

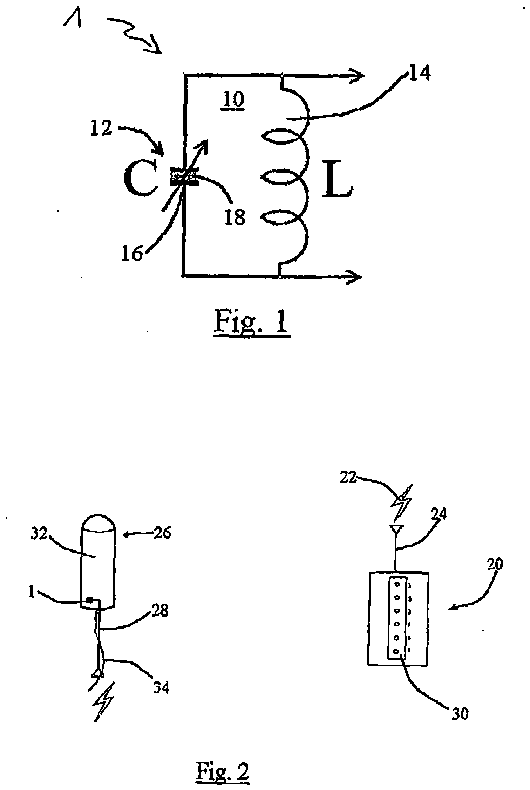

In the embodiment of the invention shown schematically in Fig. 1, the sensor 1 is an absorptive wave meter and includes an electrical circuit 10 formed of a variable capacitor 12 in parallel with an inductor 14. The resonance frequency of this circuit varies, depending upon the condition of the circuit and its surrounding environment. The circuit has a base resonance frequency. The plates 16 of the capacitor move apart or closer depending on the condition of absorbent material in the surrounding environment. Such a sensor element changes in response to the amount of dampness, thereby causing a change in the resonance frequency. The capacitor 12 has plates 16 that move apart as absorbent material 18 located between them absorbs fluid and swells. Alternatively, the plates 16 move closer together if absorbent material 18 located between them is susceptible to wet collapse or shrinkage. Alternately, the fluid itself may change the dielectric nature between the plates 16 without causing the plates to move. This change in the dielectric properties will also cause the resonance frequency of the circuit to change.

In contrast to the capacitor 12, inductor 14 is the fixed device in the circuit 10. Preferably, the inductor 14 is a length of coiled wire in a fixed configuration.

As shown in Fig. 2, a transmitter in the remote device 20 transmits a low-level signal 22 through antenna 24, which traverses from a low frequency band to a high frequency band. When the frequency of the transmission matches the resonance frequency of the sensor 1, the sensor 1 absorbs some or all of the electromagnetic energy being transmitted resulting in a drop in signal strength. The receiver of the remote device 20 can detect this drop in strength. Upon insertion of the disposable article (e.g., a tampon 26) into a body cavity (not shown), the remote device 20 can determine a base resonance frequency. Upon exposure of the sensor 1 to fluid, the resonance frequency changes. This frequency change from the base level can allow the user to determine the amount of fluid present in the tampon 26. Interrogation may occur through the body. An antenna 28 may be coupled to the sensor 1 to enhance its performance.

The user will be able to obtain information from the remote device 20 via the reporting element 30. The reporting element 30 may report information through any sensory means, including without limitation, tactile (e.g., vibrations), audio (e.g., tones or a buzzer), visual (e.g., lights as shown in Fig. 2, color change, alphanumeric display), and the like. The information can be provided continually or as prompted by the user. It can also provide information regarding the moisture change over time at any particular sensor 1 and the rate of absorption thereat to allow measurement of the performance of the tampon 26.

The absorbent material 18 can have dielectric properties that change with absorbed liquid (such as chemical salts and other ionic materials), hydrogel or superabsorbent material (such as hydrolyzed starch-acrylonitrile copolymer graft copolymer, a neutralized starch-acrylic acid graft copolymer, a saponified acrylic acid ester-vinyl acetate copolymer, a hydrolyzed acrylonitrile copolymer or acrylamide copolymer, a modified cross-liked polyvinyl alcohol, a neutralized self-crosslinking polyacrylic acid, a crosslinked polyacrylate salt, carboxylated celluslose, and a neutralized crosslinked isobutylene-malsic anhydride copolymer), absorbent fibrous or foamed structure, and the like. It may be necessary to isolate the absorbent material from the electrical components of the circuit in or to allow the circuit to function.

If hydrogel material is used as the absorbent material 18 between plates 16, fluid may be initially absorbed into the hydrogel material as the tampon is exposed to fluid. As the hydrogel continues to absorb fluid and swell, the resonance frequency of the sensor changes over the time period. This change in resonance frequency represents the flux of fluid over time. At a certain fluid point, the sensor will show that the material 18 is approaching saturation.

Users may be able to track the history of change using a small remote computer that may also be contained in the remote device 20. In this manner, the user can determine the tampon absorption threshold prior to tampon failure. By using the same tampon size or absorbency, the user can set "default" values. Additionally, particular tampons may have absorption patterns that can lead to a prediction of failure. An absorption pattern may include the rate of absorption and the rate of fluid approach to the tampon.

At least one sensor 1 is positioned in a disposable article. The location of the sensors will depend on what is to be monitored. For example, if the sensors are within a tampon used during light flow days, the user may be concerned with detecting by-pass leakage in which fluid manages to travel from the cervix down vaginal walls toward the introitis without contacting the sides of the absorbent device and being absorbed therein. In this case, at least one sensor may be placed near the outer surface or cover 32 of the tampon near the withdrawal string 34 which is normally close to the introitus.

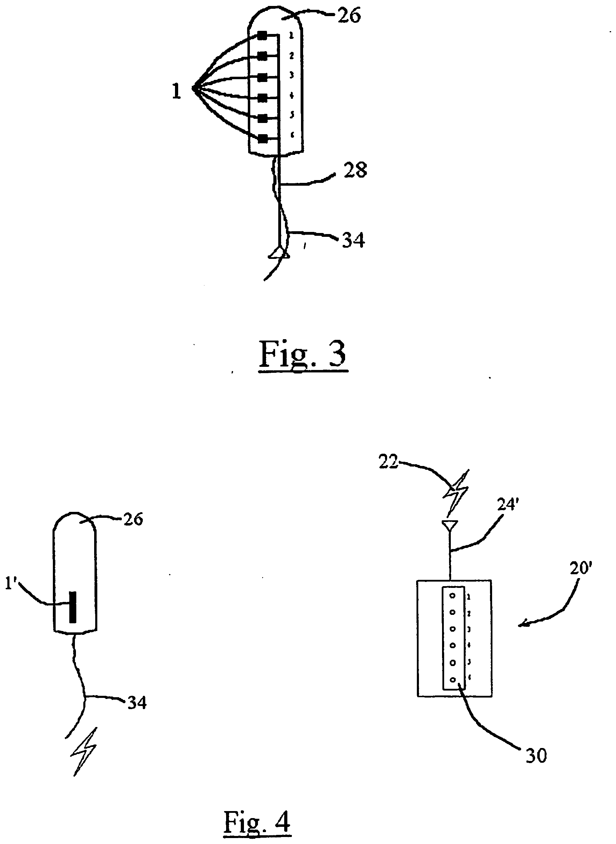

While the invention has been described above having one sensor, it can also incorporate multiple sensors distributed about the absorbent body. Sensors 1 can also be placed in arrays (linear, vertical or radial array), randomly deposited in the absorbent core or adjacent the cover 32. This would allow one to monitor the rate of fluid uptake and the location of the fluid within the absorbent body, and relevant data could be provided to the computer. An example of such a system having an array of sensors 1 is shown in Fig. 3.

An alternative system is shown in Fig. 4. The system includes a disposable article (e.g., a tampon 26) having at least one sensor 1' contained therein and a remote device 20'. Sensor 1' has alterable magnetic properties based upon moisture level therein. For example, the sensor's metallic size and/or appearance may change in the presence of liquids to provide an altered target for the remote device 20'. The remote device 20' has an oscillator that produces a low frequency magnetic field, below 10 MHz, and usually below 300 kHz.

In use, tampon 26 is inserted into a body cavity, and the remote device 20' having a transmitter coupled to the oscillator is held outside the body. The transmitter delivers the low frequency signal to the antenna 24' (usually a coil) to generate a magnetic field having a polarity. The magnetic field penetrates the body to the tampon 26. Antenna 24' may be made from any material including foilized film or metallic slivers embedded within a string. As the magnetic target (sensor 1') enters the magnetic field, the field will induce an electrical current in the sensor 1'. This current flow inside the sensor 1' in turn produces its own magnetic field, with a polarity that tends to be pointed opposite to the transmitted magnetic field. This field produces a signal that is detectable by the receiver.

The resulting received signal will usually appear delayed when compared to the transmitted signal. This delay ("phase shift") is due to the tendancy of conductors to impede the flow of current (resistance) and to impede changes in the flow of current (inductance). The largest phase shift will occur for metal objects that are primarily inductive; large, thick objects made from excellent conductors like gold, silver, and copper. Smaller phase shifts are typical for objects which are primarily resistive; smaller, thinner objects, or those composed of loss conductive materials. The phase shift can then be reported via reporting element 30 to the user as described above.

The disposable article may initially absorb a manor amount of fluids upon insertion into the body. It is preferred that this minor amount does not cause a change in the state of the sensor 1. It is preferred that a sensor change would be caused by sufficient fluid migrating or penetrating in a manner predetermined to be indicative of the approach of failure of the absorbent device, e.g., a saturation point of the absorbent material 18 or a significant proportion of the absorbent capacity of the absorbent material 18. It will be recognized that during manufacture, shipping, and storage of the disposable article, the absorbent material 18 will have an acceptable range of relative humidity (RH). This ambient RH should not affect the performance of the sensor 1.

The specification and embodimemts above are presented to aid in the complete and non-limiting understanding of the invention disclosed herein. Since many variations and embodiments of the invention can be made without departing from its scope, this invention resides in the claims hereinafter appended.

## Claims

1. A system for detecting wetness in an absorbent article, the system comprising:
an absorbent structure; at least one sensor (1) in contact with the absorbent structure, which sensor (1) is adapted to provide a variable electrical output dependent upon an amount of aqueous liquid associated with the absorbent structure; a remote interrogating device (20) capable of detecting changes in the variable electrical output of the at least one sensor (1); and a reporting element (30) coupled to the interrogating device (20) **characterized in that**,
the at least one sensor (1) comprises an electrical circuit (10) having an inductor (14) and a variable capacitor (12) electrically connected in parallel, the capacitor (12) has plates (16) that are adapted to move apart when absorbent material (18) located between them absorbs fluid and swells, and that are adapted to move closer together when absorbent material (18) located between them is susceptible to wet collapse or shrinkage, wherein the variable capacitor (12) has a capacitance that changes with the amount of aqueous liquid associated with the absorbent reservoir, wherein the electrical circuit (10) has a resonance frequency that changes with the amount of aqueous liquid associated with the absorbent reservoir, and wherein the interrogating device is (20) capable of detecting a resonance frequency of the electrical circuit (10).

2. The system of claim 1 wherein
the absorbent structure is associated with a non-absorbent structure.

3. The system of claim 1 wherein
the at least one sensor (1) comprises a plurality of sensors.

4. The system of claim 3 wherein
a first sensor is associated with a portion of the absorbent structure directed away from the source of bodily fluids.

5. The system of claim 4 wherein
the plurality of sensors comprises a second sensor associated with a portion of the absorbent structure directed toward the source of bodily fluids.

6. The system of claim 3 wherein
the remote interrogating device is capable of discriminating between each of the plurality of sensors.

7. The system of claim 1 wherein
the absorbent structure is a tampon.

8. A method of controlling liquid bodily exudates comprising the steps of:
a) placing a first disposable article in proximity to a source of liquid bodily exudates, the disposable article including a system for detecting wetness according to any of the aforementioned claims
b) allowing the absorbent structure to absorb liquid bodily exudates;
c) transmitting a signal from a remote interrogating device;
d) detecting a change in the variable electrical output of the at least one sensor; and
e) reporting information based upon the signal detected.

9. The method of claim 8 wherein
the step of detecting a change in the variable electrical output comprises detecting a change in the signal transmitted from the remote interrogating device, the change being related to the variable electrical output of the at least one sensor.

10. The method of claim 8 wherein
the step of detecting a change in the variable electrical output comprises transmitting signals of varying frequency to determine the resonance frequency of the at least one sensor.

11. The method of claim 8 wherein
the at least one sensor comprises a plurality of sensors.

12. The method of claim 11 wherein
a first sensor is associated with a portion of the absorbent structure directed away from the source of liquid bodily exudates.

13. The method of claim 12 wherein
the plurality of sensors comprises a second sensor associated with a portion of the absorbent structure directed toward the source of liquid bodily exudates.

14. The method of claim 11 further comprising
the step of discriminating between the change in signal transmitted from the remote interrogating device related to the variable electrical output of each of the plurality of sensors to provide information about each of the plurality of sensors.

15. The method of claim 14 further comprising
the step of analyzing the information related to each of the plurality of sensors to predict leakage of liquid bodily exudates past the disposable article.

16. The method of claim 8 wherein
the step of detecting a change in the variable electrical output comprises transmitting signal having a frequency of less than about 300 kHz to determine a phase shift caused by magnetic properties of the at least one sensor.

## Patentansprüche

1. System zum Erfassen von Feuchtigkeit in einem absorbierenden Artikel, umfassend: eine absorbierende Struktur; mindestens einen mit der absorbierenden Struktur in Kontakt stehenden Sensor (1), wobei der Sensor (1) so gestaltet ist, dass er eine variable elektrische Ausgabe in Abhängigkeit von einer Menge an wässriger Flüssigkeit, die der absorbierenden Struktur zugeordnet ist, bereitstellt; eine entfernte Abfragevorrichtung (20), die in der Lage ist, Veränderungen der variablen elektrischen Ausgabe des mindestens einen Sensors (1) zu erfassen; und ein mit der Abfragevorrichtung (20) gekoppeltes Meldeelement (30), **dadurch gekennzeichnet, dass**
der mindestens eine Sensor (1) einen elektrischen Stromkreis (10) mit einem Induktor (14) und einem elektrisch parallel geschalteten variablen Kondensator (12) umfasst, wobei der Kondensator (12) Platten (16) aufweist, die so gestaltet sind, dass sie sich voneinander wegbewegen, wenn zwischen ihnen angeordnetes absorbierendes Material (18) Flüssigkeit absorbiert und aufquillt, und die so gestaltet sind, dass sie sich aufeinander zubewegen, wenn zwischen ihnen angeordnetes absorbierendes Material (18) unter Feuchtigkeit dafür anfällig ist, zusammenzufallen oder zu schrumpfen, wobei der variable Kondensator (12) eine Kapazität aufweist, die sich mit der Menge an wässriger Flüssigkeit, die dem Absorptionsreservoir zugeordnet ist, verändert, wobei der elektrische Stromkreis (10) eine Resonanzfrequenz aufweist, die sich mit der Menge an wässriger Flüssigkeit, die dem Absorptionsreservoir zugeordnet ist, verändert, und wobei die Abfragevorrichtung (20) in der Lage ist, eine Resonanzfrequenz des elektrischen Stromkreises (10) zu erfassen.

2. System nach Anspruch 1, wobei die absorbierende Struktur einer nichtabsorbierenden Struktur zugeordnet ist.

3. System nach Anspruch 1, wobei der mindestens eine Sensor (1) eine Mehrzahl von Sensoren umfasst.

4. System nach Anspruch 3, wobei ein erster Sensor einem der Quelle von Körperflüssigkeiten abgewandten Abschnitt der absorbierenden Struktur zugeordnet ist.

5. System nach Anspruch 4, wobei die Mehrzahl von Sensoren einen zweiten Sensor umfasst, der einem der Quelle von Körperflüssigkeiten zugewandten Abschnitt der absorbierenden Struktur zugeordnet ist.

6. System nach Anspruch 3, wobei die entfernte Abfragevorrichtung in der Lage ist, zwischen jedem aus der Mehrzahl von Sensoren zu unterscheiden.

7. System nach Anspruch 1, wobei die absorbierende Struktur ein Tampon ist.

8. Verfahren zum Kontrollieren flüssiger Körperexsudate, umfassend folgende Schritte:
a) Anordnen eines ersten Einwegartikels in der Nähe einer Quelle flüssiger Körperexsudate, wobei der Einwegartikel ein System zum Erkennen von Feuchtigkeit nach einem der vorherigen Ansprüche enthält;
b) Zulassen, dass die absorbierende Struktur flüssige Körperexsudate absorbiert;
c) Übertragen eines Signals von einer entfernter Abfragevorrichtung;
d) Erfassen einer Veränderung der variablen elektrischen Ausgabe des mindestens einen Sensors; und
e) Melden von Informationen auf Grundlage des erfassten Signals.

9. Verfahren nach Anspruch 8, wobei der Schritt des Erfassens einer Veränderung der variablen elektrischen Ausgabe das Erfassen einer Veränderung des von der entfernten Abfragevorrichtung übertragenen Signals umfasst, wobei die Veränderung zur variablen elektrischen Ausgabe des mindestens einen Sensors in Beziehung steht.

10. Verfahren nach Anspruch 8, wobei der Schritt des Erfassens einer Veränderung der variablen elektrischen Ausgabe das Übertragen von Signalen variierender Frequenz umfasst, um die Resonanzfrequenz des mindestens einen Sensors zu bestimmen.

11. Verfahren nach Anspruch 8, wobei der mindestens eine Sensor eine Mehrzahl von Sensoren umfasst.

12. Verfahren nach Anspruch 11, wobei ein erster Sensor einem von der Quelle flüssiger Körperexsudate abgewandten Abschnitt der absorbierenden Struktur zugeordnet ist.

13. Verfahren nach Anspruch 12, wobei die Mehrzahl von Sensoren einen zweiten Sensor umfasst, der einem der Quelle flüssiger Körperexsudate zugewandten Abschnitt der absorbierenden Struktur zugeordnet ist.

14. Verfahren nach Anspruch 11, ferner umfassend den Schritt des Unterscheidens zwischen der Veränderung des von der entfernten Abfragevorrichtung übertragenen Signals bezüglich der variablen elektrischen Ausgabe jedes aus der Mehrzahl von Sensoren, um Informationen über jeden aus der Mehrzahl von Sensoren bereitzustellen.

15. Verfahren nach Anspruch 14, ferner umfassend den Schritt des Analysierens der Informationen bezüglich jedes der Mehrzahl von Sensoren, um das Auslaufen flüssiger Kör-perexsudate aus dem Einwegartikel zu prognostizieren.

16. Verfahren nach Anspruch 8, wobei der Schritt des Erfassens einer Veränderung der variablen elektrischen Ausgabe das Übertragen eines Signals mit einer Frequenz von weniger als etwa 300 kHz umfasst, um eine durch magnetische Eigenschaften des mindestens einen Sensors hervorgerufene Phasenverschiebung zu bestimmen.

## Revendications

1. Système de détection d'humidité contenue dans un article absorbant, comprenant: une structure absorbante; au moins un capteur (1) étant en contact avec la structure absorbante, le capteur (1) étant adapté à fournir une sortie électrique variable en fonction d'une quantité de liquide aqueux associée à la structure absorbante; un dispositif d'interrogation à distance (20) étant capable de détecter des changements de la sortie électrique variable de l'au moins un capteur (1); et un élément de rapport (30) couplé au dispositif d'interrogation (20), **caractérisé en ce que**
l'au moins un capteur (1) comprend un circuit électrique (10) ayant une bobine d'inductance (14) et un condensateur variable (12) connectés électriquement en parallèle, le condensateur (12) ayant des plaques (16) adaptées à s'écarter quand du matériel absorbant (18) disposé entre eux absorbe du fluide et gonfle, et adaptées à se rapprocher quand du matériel absorbant (18) disposé entre eux est susceptible de d'affaisser ou de se rétrécir sous l'influence de l'humidité, le condensateur variable (12) ayant une capacité qui change par rapport à la quantité de liquide aqueux associée au réservoir absorbant, le circuit électrique (10) ayant une fréquence de résonnance qui change par rapport à la quantité de liquide aqueux associée au réservoir absorbant et le dispositif d'interrogation (20) étant capable de détecter une fréquence de résonnance du circuit électrique (10).

2. Système selon la revendication 1, la structure absorbante étant associée à une structure non absorbante.

3. Système selon la revendication 1, l'au moins un capteur (1) comprenant une pluralité de capteurs.

4. Système selon la revendication 3, un premier capteur étant associé à une partie de la structure absorbante orientée à l'opposé de la source de fluides corporels.

5. Système selon la revendication 4, la pluralité de capteurs comprenant un deuxième capteur associé à une partie de la structure orientée vers la source de fluides corporels.

6. Système selon la revendication 3, le dispositif d'interrogation à distance étant capable de distinguer chacun de la pluralité de capteurs.

7. Système selon la revendication 1, la structure absorbante étant un tampon.

8. Procédé de contrôle de sécrétions corporelles liquides, comprenant les étapes suivantes:
a) placer un premier article jetable à proximité d'une source de sécrétions corporelles liquides, l'article jetable contient un système de détection d'humidité selon une des revendications précédentes;
b) permettre à la structure absorbante d'absorber des sécrétions corporelles liquides;
c) transmettre un signal d'un dispositif d'interrogation à distance;
d) détecter un changement de la sortie électrique variable de l'au moins un capteur; et
e) reporter des informations sur la base du signal détecté.

9. Procédé selon la revendication 8, l'étape de détection d'un changement de la sortie électrique variable comprenant la détection d'un changement du signal transmis du dispositif d'interrogation, le changement étant lié à la sortie électrique variable de l'au moins un capteur.

10. Procédé selon la revendication 8, l'étape de détection d'un changement de la sortie électrique variable comprenant la transmission de signaux de fréquence variable afin de déterminer la fréquence de résonnance de l'au moins un capteur.

11. Procédé selon la revendication 8, l'au moins un capteur comprenant une pluralité de capteurs.

12. Procédé selon la revendication 11, un premier capteur étant associé à une partie de la structure absorbante orientée à l'opposé de la source de sécrétions corporelles liquides.

13. Procédé selon la revendication 12, la pluralité de capteurs comprenant un deuxième capteur associé à une partie la structure absorbante orientée vers la source de sécrétions corporelles liquides.

14. Procédé selon la revendication 11, comprenant en outre l'étape de distinction du changement du signal transmis du dispositif d'interrogation par rapport à la sortie électrique variable de chacun de la pluralité de capteurs afin de fournir des informations sur chacun de la pluralité de capteurs.

15. Procédé selon la revendication 14, comprenant en outre l'étape d'analyse des informations se rapportant à chacun de la pluralité de capteurs afin de prédire une fuite de sécrétions corporelles liquides de l'article jetable.

16. Procédé selon la revendication 8, l'étape de détection d'un changement de la sortie électrique variable comprenant la transmission d'un signal ayant une fréquence inférieure à 300 kHz environ afin de déterminer un décalage de phase causé par les propriétés magnétiques de l'au moins un capteur.
